# EUROPEAN PATENT SPECIFICATION

(11) **EP 0 420 763 B1**
(45) Date of publication and mention of the grant of the patent: **28.07.1999**
(21) Application number: 90402693.7
(22) Date of filing: 28.09.1990
(51) Int. Cl.: C07D 239/60, C07D 239/54, C07D 239/56, C07D 239/47, A61K 31/505

(54) **6-Substituted acyclopyrimidine nucleoside derivatives and antiviral agent containing the same as active ingredient thereof**
Derivate von 6-substituierten azyklischen Pyrimidin-Nukleosiden und diese als Wirkstoff enthaltende antivirale Mittel
Dérivés de nucléosides d'acyclopyrimidines 6-substituées et agent antiviral les contenant comme ingrédients actifs

(30) Priority: 29.09.1989 JP 25453189; 09.03.1990 JP 5970090; 27.07.1990 JP 20089590
(43) Date of publication of application: 03.04.1991
(60) Divisional application: 97110658.8
(73) Proprietor: Mitsubishi Chemical Corporation, Chiyoda-ku Tokyo (JP)
(72) Inventor: Miyasaka, Tadashi, Yokohama-shi, Kanagawa-ken (JP); Tanaka, Hiromichi, Yokohama-shi, Kanagawa-ken (JP); De Clercq, Erik Desire Alice, B-3000 Leuven (BE); Baba, Masanori, Fukushima-shi, Fukushima-ken (JP); Walker, Richard Thomas, Birmingham (GB); Ubasawa, Masaru, Yokohama-shi, Kanagawa-ken (JP)
(74) Representative: Gutmann, Ernest

(56) References cited:
- EP-A- 0 371 139
- WO-A-83/02891
- DE-A- 2 126 148
- DE-A- 2 142 317
- US-A- 3 497 515
- TETRAHEDRON LETTERS, vol. 27, no. 1, January 1986, pages 113-116, Pergamon Press Ltd, Oxford, GB; I.T. KAY et al.: "A new synthesis of phenyl ketones by intramolecular "deoxybenzoylation" of enols and phenols"
- TETRAHEDRON, vol. 39, no. 23, December 1983, pages 3919-3921, Pergamon Press Ltd, Oxford, GB; M. SAKO et al.: "Reductive debromination of 5-bromouracils by 1-benzyl-1,4-dihydronicotinamide"
- JOURNAL OF HETEROCYCLIC CHEMISTRY, vol. 19, no. 2, March-April 1982, pages 301-304; F. YONEDA et al.: "Synthesis of 2H-chromeno[2,3-d]pyrimidine-2,4(3H)-diones (10-oxa-5-deazaflavins)"
- JOURNAL OF MEDICINAL CHEMISTRY, vol. 10, no. 3, May 1967, pages 304-311, Washington, DC, US; B.R. BAKER et al.: "Irreversible enzyme inhibitors. LXXVII"
- JOURNAL OF MEDICINAL CHEMISTRY, vol. 10, no. 3, May 1967, pages 316-320, Washington, DC, US; B.R. BAKER et al.: "Irreversible enzyme inhibitors. LXXX"
- JOURNAL OF MEDICINAL CHEMISTRY, vol. 19, no. 1, January 1976, pages 71-98, Washington, DC, US; M. YOSHIMOTO et al.: "Correlation analysis of Baker's studies on enzyme inhibition. 2."
- CHEMICAL ABSTRACTS, vol. 111, no. 17, 23rd October 1989, page 728, abstract no. 153828m, Columbus, Ohio, US; & JP-A-63 290 867 (SDS BIOTECH.) 28-11-1988
- CHEMICAL ABSTRACTS, vol. 106, no. 3, 19th January 1987, page 613, abstract no. 18608w, Columbus, Ohio, US; & JP-A-61 205 261 (SAGAMI CHEMICAL RESEARCH CENTRE) 11-09-1987

## Description

### FIELD OF THE INVENTION

The present invention relates to novel 6-substituted acyclopyrimidine derivatives and antiviral agents containing the derivative as the active ingredients.

### BACKGROUND OF THE INVENTION

Infectious diseases caused by human acquired immunodeficiency virus (HIV), which is a type of retrovirus, have recently become a serious social problem. A compound of 3'-deoxy-3'-azidothymidine is known as a nucleoside compound used in the clinical treatment for diseases caused by HIV-infection. However, this compound has side-effects since it also exhibits considerable strong toxicity in the host cells.

Although some 2',3'-dideoxyribonucleosides are known as nucleoside compounds exhibiting an anti-viral activity, it is still necessary to develop a substance possessing a higher activity and lower toxicity to the host cell (Hiroaki Mitsuya, Bodily Defense, Vol. 4, pp.213 - 223 (1987)).

On the other hand, various acyclonucleoside compounds have been synthesized since Acyclovir (acycloguanosine) was developed as an antiviral substance effective against herpes virus (C.K. Chu and S.J. Culter, J. Heterocyclic Chem., 23, p.289 (1986)). However, no acyclonucleoside compound having a sufficient activity especially against retroviruses has yet been discovered.

We have focussed our attention on 6-substituted acyclopyrimidine nucleoside compounds and have synthesized various novel 6-substituted acyclopyrimidine nucleoside derivatives and screened those compounds to detect effective antiviral agents, especially to the retrovirus, in order to provide antiviral agents exhibiting an effective activity particularly against retroviruses.

Some 6-substituted acyclopyrimidine nucleoside compounds such as 6-fluoro substituted derivatives, 6-alkylamino substituted derivatives (DD-A-232492) and 6-methyl substituted derivatives (C.A. 107, 129717w (1987)) are known; however, the antiviral activity of these compounds has not been described.

EP 371139 discloses 6-substituted acyclopyrimidine derivatives in which the substituant of the nitrogen atom in position 1 carries a hydroxyalkyl group of which alkyl portion may contain an oxygen atom and which have antiviral activity. The inhibitory activity for HIV infection of some of these derivatives has been determined by the following test.

Inhibitory activity for HIV infection

In RPMI 1640 DM culture medium containing 20 mM of Hepes buffer solution, 10 % fetal bovine serum and 20 g/ml of gentamycin, 3 x 10⁴ MT-4 cells (human T cell clone which is destroyed by the infection of HIV) were infected with HIV in an amount of 100 times as large as expected to cause 50 % infection of the cells. Immediately thereafter, a predetermined amount of sample was added to the culture medium using 50 mg/ml sample solutions in dimethyl sulfoxide and the cells were cultured at 37°C.

After 5 days of incubation, the number of existing cells was counted to determine the concentration of the compound for preventing the death of 50 % of the MT-4 cells. Separately, MT-4 cells were cultured in the same way as above except that they were not infected with HIV to determine the concentration of the compound at which 50 % of the MT-4 cells were destroyed.

As a result of our researches for compounds exhibiting an effective antiviral activity, particularly anti-retroviral activity, we found that specific 6-substituted pyrimidine nucleoside compounds according to the invention satisfy the above demand to achieve the present invention.

The present invention concerns 6-substituted acyclopyrimidine nucleoside derivatives represented by the following general formula I; 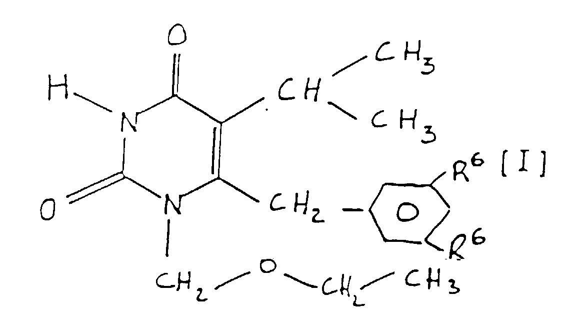 wherein R⁶ are both either H or CH₃, pharmaceutically acceptable salts thereof and antiviral agents containing the derivative or the salt thereof as an active ingredient.

The 6-substituted acyclopyrimidine nucleoside derivatives according to the invention are represented by the general formula I.

Examples of the compounds according to the present invention are listed in Table 1 below.

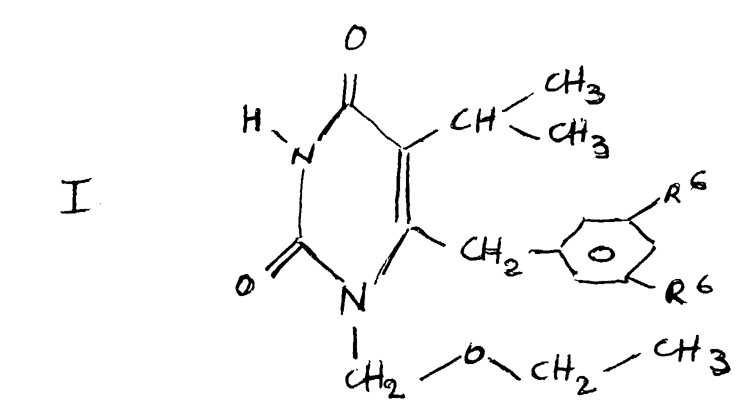

**Table 1**

| compound N° | R⁶ |
|---|---|
| 1 | H |
| 2 | CH₃ |

The compounds according to the invention of the formula I may be prepared in accordance with the following reaction formula (1), (2), (3) or (4): 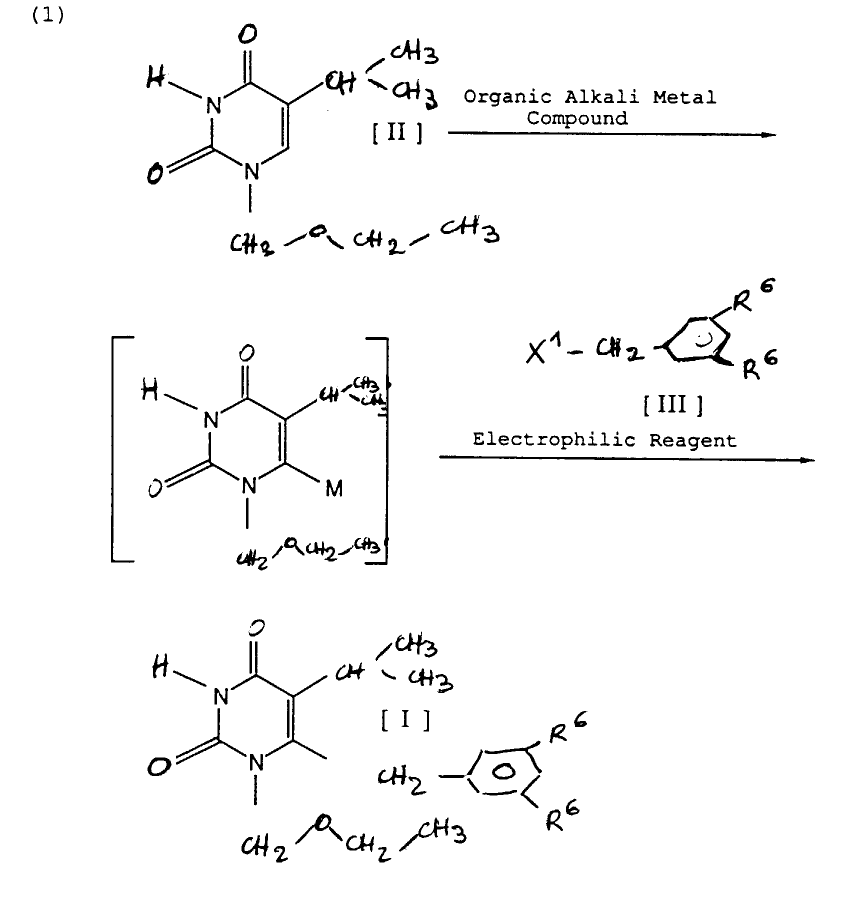 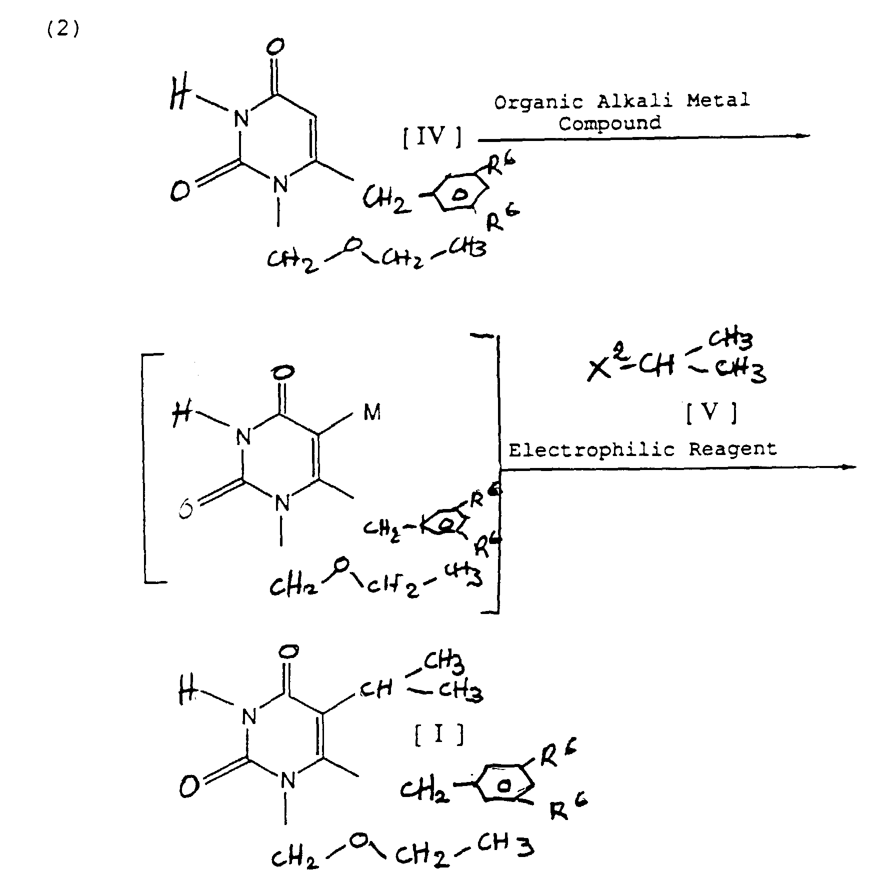 wherein R⁶ has the same meaning as defined hereinbefore, X¹ and X² represent a halogen atom or the like, and M represents an alkaline metal.

Firstly, the compound of the formula II or IV is treated with an organic alkali metal compound in an ether solvent such as diethyl ether and tetrahydrofuran at a temperature of -80 to -10°C for 0.2 to 10 hours.

Examples of the organic alkali metal compound include potassium bistrimethylsilylamide, sodium bistrimethylsilylamide and lithium alkylamide, and particularly preferred compounds among those are lithium diisopropylamide (LDA) and lithium 2,2,6,6-tetramethylpiperidide (LTMP). Such lithium alkylamides are preferably prepared immediately before the reaction. For example, lithium dialkylamide may be prepared by reacting a secondary amine such as diisopropylamine with an alkyl lithium such as n-butyl lithium in a solvent such as diethyl ether, dioxane, tetrahydrofuran and dimethoxyethane with stirring under the atmosphere of an inert gas such as argon at -80°C to -10°C for 0.2 to 5 hours.

The organic alkali metal compound is usually used in an amount of 1 to 5 moles per mole of the compound of the general formula II or IV.

Then, the electrophilic reagent of the general formula III or V is added to the reaction mixture in a ratio of about 1 to 5 moles to the compound of the general formula II or IV to allow the reaction under the same condition as in the reaction with the organic alkali metal compound.

The electrophilic reagent should have a group -CH(CH₃)₂or 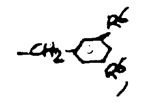 R⁶ being defined above, and examples of this reagent includes various alkyl halides, aralkyl halides such as benzyl bromide and the like.

The compounds of the general formula II can be prepared by a conventional method.

The compounds of the general formula IV can be prepared in accordance with the reaction formula (1) above where H replaces 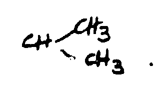 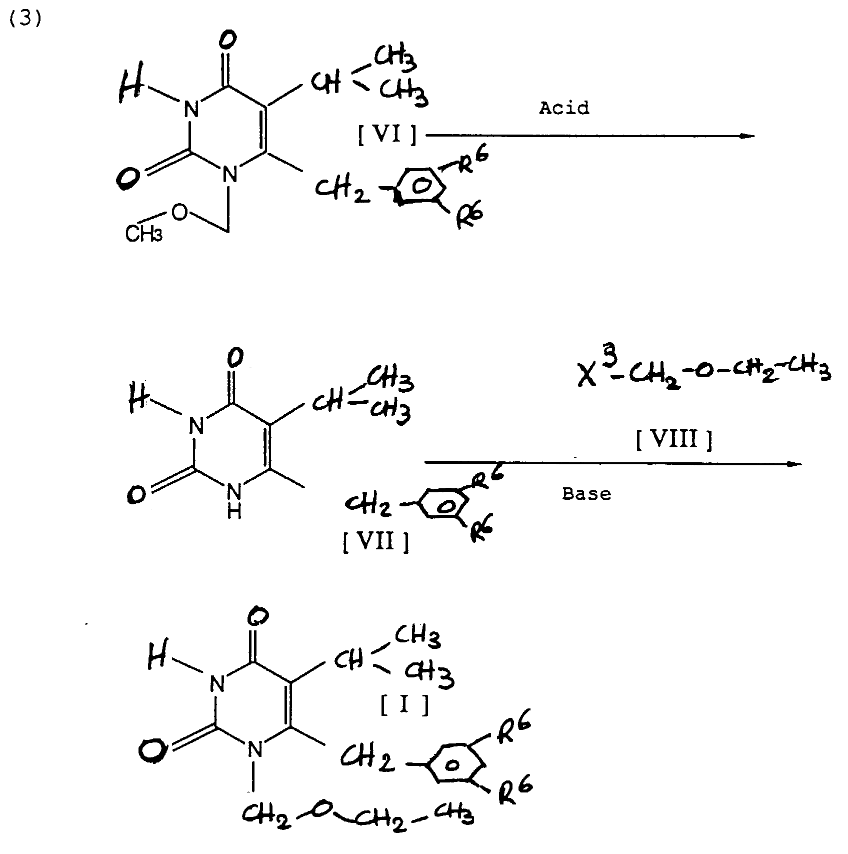 wherein R⁶ has the same meaning as defined hereinbefore and X³ represents a halogen atom such as chlorine, bromine and iodine or sulfonyloxy group such as toluenesulfonyloxy and mesyloxy groups.

The compounds of the general formula VI are treated with an acid such as hydrochloric acid and bromic acid in a suitable solvent, for example, an alcohol such as methanol and ethanol and water at an appropriate temperature of from room temperature to 100°C to obtain the compounds of the general formula VII.

Then, the compounds of the general formula VII are reacted with the compounds of the general formula VIII in a suitable solvent such as dimethylformamide, dimethyl sulfoxide, acetonitrile and tetrahydrofuran in the presence of a suitable base such as sodium hydride, sodium alkoxide, potassium alkoxide, potassium carbonate and sodium carbonate at a temperature of from ambient temperature to the boiling point of the solvent to obtain the compounds of the general formula I.

The starting compounds represented by the general formula VI can be prepared in accordance with the reaction formula (1) or (2).

The 6-benzyl substituted derivatives of the invention may be prepared in accordance with the reaction formula (4) below: 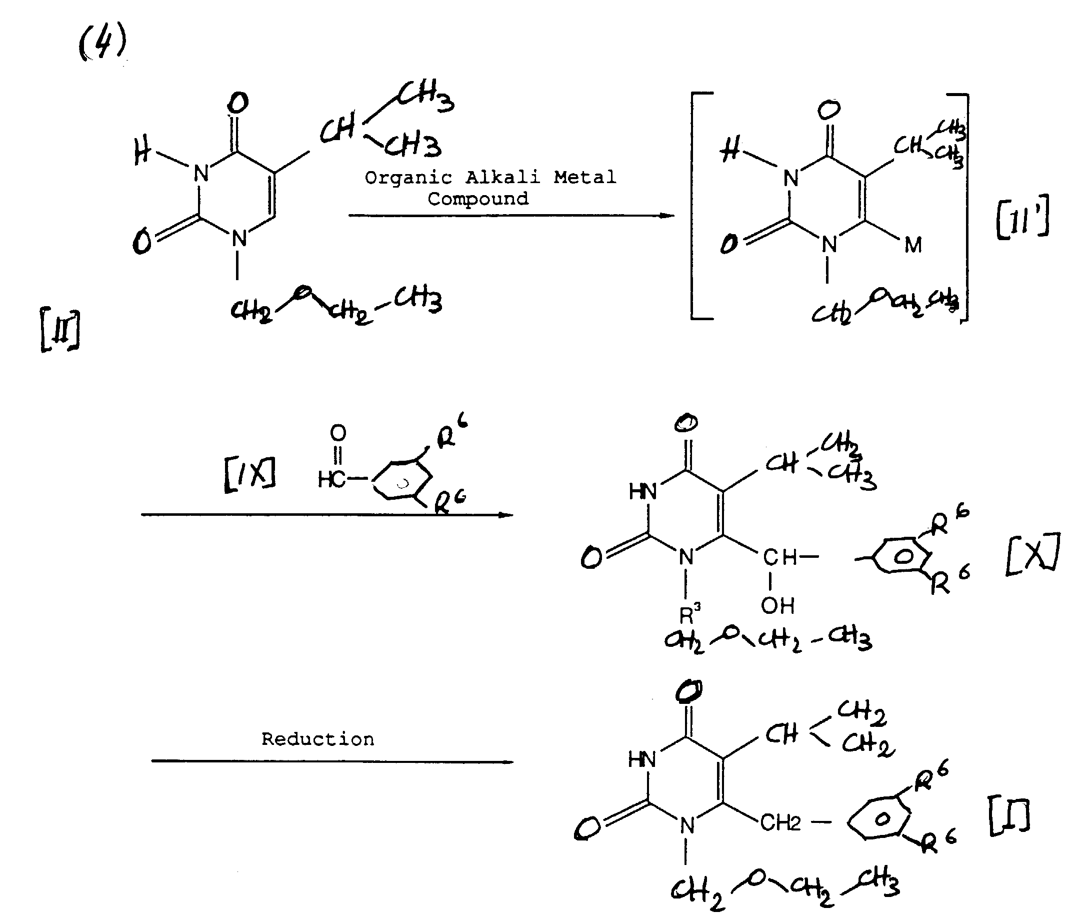 wherein the symbols R⁶ has the same meanings as defined hereinbefore.

In the reactions of the formula (4), intermediate compounds are prepared in the same way as the reactions of the formula (1) using OHC- 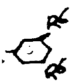 instead of and the intermediate compounds are reduced by a 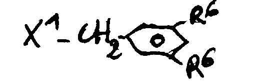 suitable reducing agent to convert the hydroxyl group into a hydrogen atom. The reduction can be carried out by using hydrogen gas in the presence of palladium/carbon or palladium hydroxide.

The compounds of the present invention obtained as described hereinbefore and represented by the formula I may be separated and purified by any of the conventional methods for the separation and purification of nucleosides, for example, recrystallization, adsorption chromatography, ion exchange chromatography and the like.

The compounds of the invention represented by the formula I may be converted into a pharmaceutically acceptable salt thereof by a conventional method. Such salt may be, for example, an alkali metal salt such as sodium or potassium salt, alkaline earth metal salt such as magnesium salt, ammonium salt or alkylammonium salt such as methylammonium, dimethylammonium, trimethylammonium, tetramethylammonium salt or the like.

The compounds according to the invention can be administered to human beings via any route, oral, rectal, parenteral or local for the prevention or treatment of the infection of viruses such as retrovirus. The administration dose of the compounds according to the invention may be determined according to age, physical condition, body weight and the like of a patient to be treated; however, a suitable daily does of the compounds is 1 to 100 mg/(body weight)kg, preferably 5 to 50 mg/(body weight)kg and it is administered in one to several times.

The compound of the invention is generally prepared in a pharmaceutical composition with a suitable carrier, excipient and other additives. Either a liquid carrier or solid carrier may be suitably used for the present antiviral agent.

Examples of the solid carrier are lactose, kaolin, sucrose, crystalline cellulose, corn starch, talc, agar, pectin, stearic acid, magnesium stearate, lecithin, sodium chloride and the like.

Examples of the liquid carrier are glycerin, peanut oil, polyvinyl pyrrolidone, olive oil, ethanol, benzyl alcohol, propylene glycol, water and the like.

The present antiviral agent may be made in various forms. For example, it may be in the form of a tablet, powder, granule, capsule, suppository, troche or the like when a solid carrier is used, and it may be also in the form of syrup, emulsion, soft gelatin capsule, cream, gel, paste, spray, injection solution, or the like when a liquid carrier is used.

The novel 6-substituted acyclopyrimidine nucleoside derivatives according to the present invention have an effective antiviral activity against viruses such as retrovirus and have a relatively low toxicity against the host cell, hence the derivatives of the invention are extremely useful as an active ingredient of antiviral agent.

Compounds No. 1 and 2 in Table 1 may be prepared similarly according to the method described in the working example below.

### Preparation of 6-benzyl-1-ethoxymethyl-5-ethyluracil

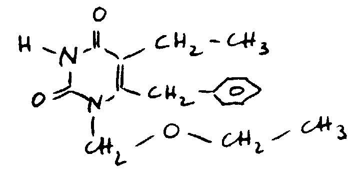

To 100 ml of methylene chloride, 5.1 g (40 mmol) of 5-ethyluracil and 22 ml (88 mmol) of bistrimethylsilylacetamide were added under a nitrogen atmosphere and stirred for 40 minutes at room temperature. To this mixture, 4.1 ml (88 mmole) of chloromethyl ethyl ether and 0.15 g (0.4 mmol) of tetrabutylammonium iodide were added and heated under reflux for 15 hours. Then, the reaction mixture was poured into 50 ml of saturated sodium bicarbonate solution carefully and filtered through Celite. The organic layer was washed with water, dried on magnesium sulfate and concentrated under reduced pressure. The residue was crystallized from ethyl acetate to obtain 6.4 g of 1-ethoxymethyl-5-ethyluracil (Yield: 81 %).

Then, 2.2 ml (4.4 mmol) of lithium diisopropylamide solution in tetrahydrofuran (2.1 M) was added to 6 ml of tetrahydrofuran under a nitrogen atmosphere at -70°C, to which a solution of 0.40 g (2.0 mmol) of 1-ethoxymethyl-5-ethyluracil in 3 ml of tetrahydrofuran was added dropwise over 15 minutes. After stirring for 1 hour at -70°C, the reaction mixture was added with a solution of 0.27 g (2.6 mmol) of benzaldehyde in 2 ml of tetrahydrofuran dropwise over 10 minutes and allowed to react for 30 minutes. The reaction mixture was added with 1 ml of acetic acid, brought to room temperature and then added with 30 ml of ethyl acetate. The mixture was washed with water (3 ml x 5) and saturated aqueous solution of sodium hydrogencarbonate (twice), dried on magnesium sulfate and concentrated under reduced pressure.

The residue was dissolved in 10 ml of ethanol, added with 20 mg of 20 % palladium hydroxide/carbon and stirred under a hydrogen atmosphere for a day at 55°C. Then, after removing the catalyst by filtration, the reaction mixture was concentrated. The residue was crystallized from hexane to obtain 0.28 g of 6-benzyl-1-ethoxymethyl-5-ethyluracil (Yield: 85 %).

## Claims

1. A 6-substituted acyclopyrimidine nucleoside derivative represented by the following general formula I; 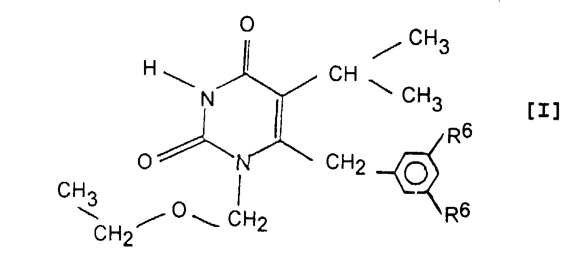 wherein:
R⁶ are both either H or CH₃,
or a pharmaceutically acceptable salt thereof.

2. An antiretroviral agent containing as an active ingredient a 6-substituted acyclopyrimidine nucleoside derivative or a pharmaceutically acceptable salt thereof according to claim 1.

3. A pharmaceutical composition containing a derivative of claim 1 or a pharmaceutically acceptable salt thereof in association with a pharmaceutical vehicle.

4. The pharmaceutical composition of claim 3 which has effective anti-retroviral activity.

5. The use of a derivative of claim 1 or a pharmaceutically acceptable salt thereof in a process for the production of a pharmaceutical composition for the treatment or prevention of diseases caused by retroviral infection, especially in human beings.

6. A process for the preparation of a compound represented by the following general formula I; 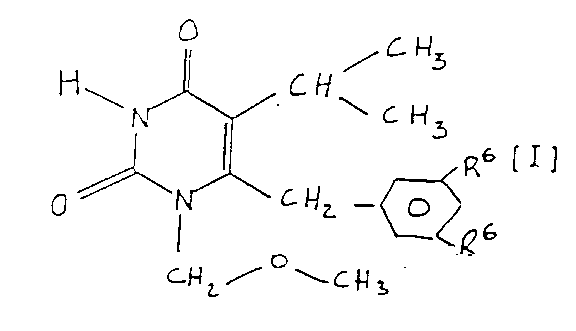 wherein:
R⁶ represent both either a hydrogen atom or CH₃ which comprises reacting a compound represented by the following general formula II; 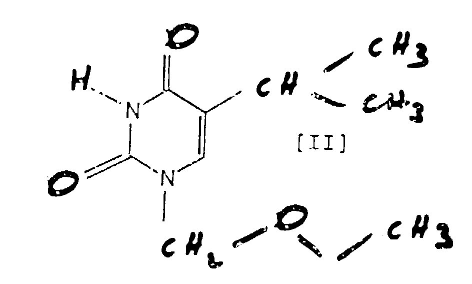 with an organic alkali metal compound to obtain a compound represented by the following general formula II'; 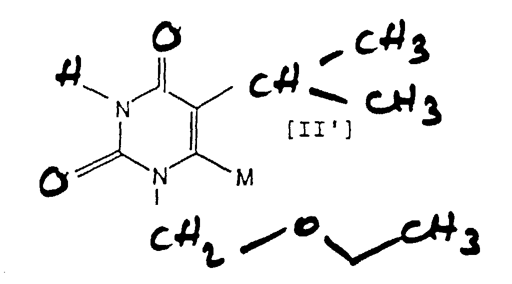 wherein M represents an alkaline metal, and then reacting the obtained compound of the formula II' with a compound represented by the following general formula III; 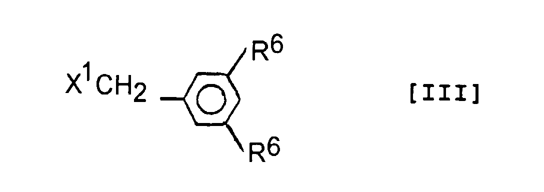 wherein R⁶ has the same meaning as defined above and X¹ represents a halogen atom, arylthio or alkoxy group, to obtain the compound of the formula I above.

7. A process for the preparation of a compound represented by the following general formula I; 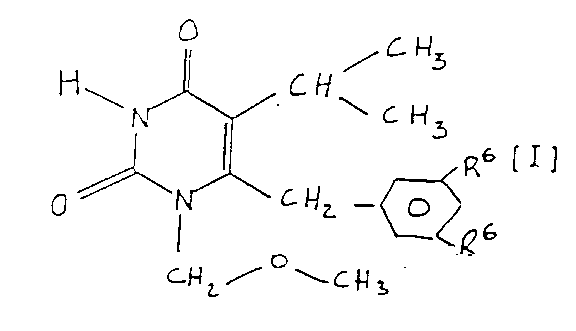 wherein R⁶ has the same meanings as defined in claim 1, which comprises reacting a compound represented by the following general formula IV; 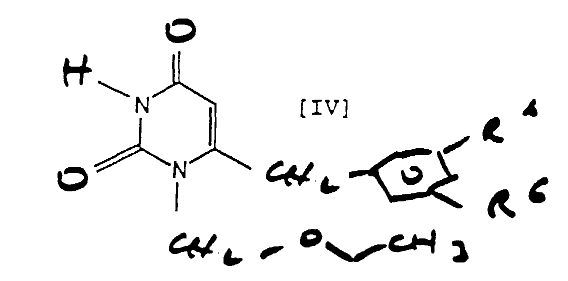 wherein R⁶ has the same meanings as defined in claim 1, with an organic alkali metal compound to obtain a compound represented by the following general formula IV'; 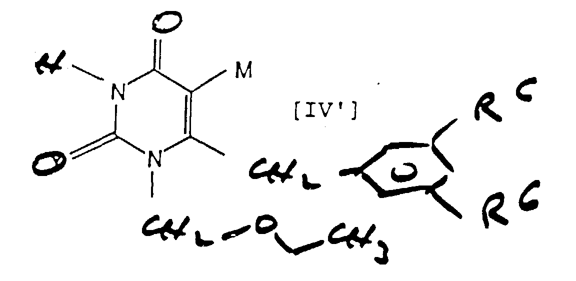 wherein R⁶ has the same meanings as defined in claim 1 and M represents an alkali metal, and then reacting the obtained compound of the formula IV' with a compound represented by the following general formula V;
[V] (CH₃)₂-CH-X²
wherein X² represents a halogen atom to obtain the compound of the formula I above.

8. A process for the preparation of a compound represented by the following general formula I; 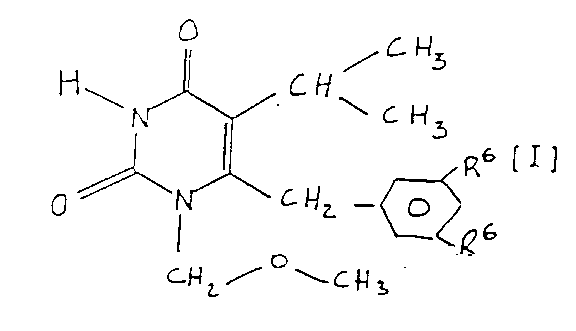 wherein R⁶, has the same meanings as defined in claim 1, which comprises reacting a compound represented by the following general formula VI; 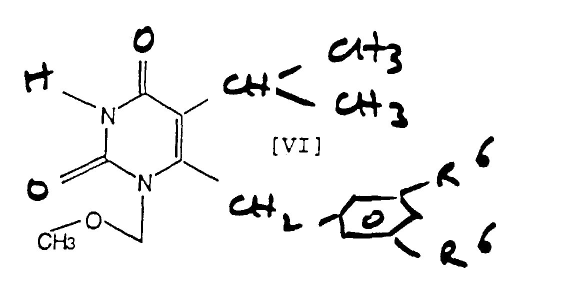 wherein R⁶, has the same meanings as defined in claim 1, with an acid to obtain a compound represented by the following general formula VII; 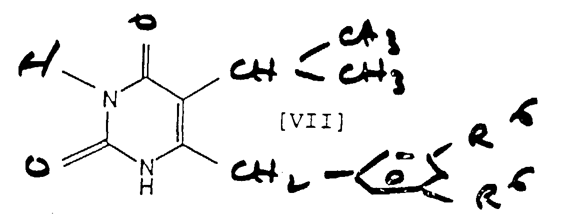 wherein R⁶, has the same meaning as defined in claim 1, and then reacting the obtained compound of the formula VII with a compound represented by the following general formula VIII;
CH₃-CH₂-O-CH₂-X³ [VIII]
wherein X³ represents a halogen atom or a sulfonyloxy group, in the presence of a base to obtain the compound of the formula I above.

9. A process for the preparation of a compound represented by the following general formula I; 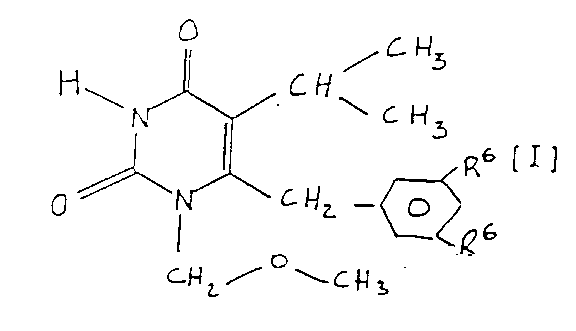 wherein R⁶ has the same meanings as defined in claim 1, which comprises reacting a compound represented by the following general formula II; 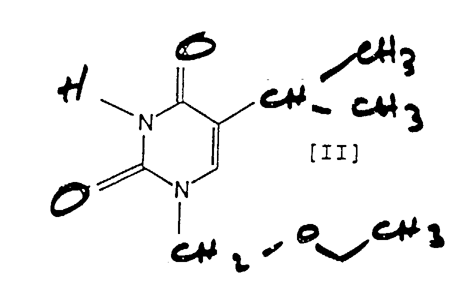 with an organic alkali metal compound to obtain a compound represented by the following general formula II'; 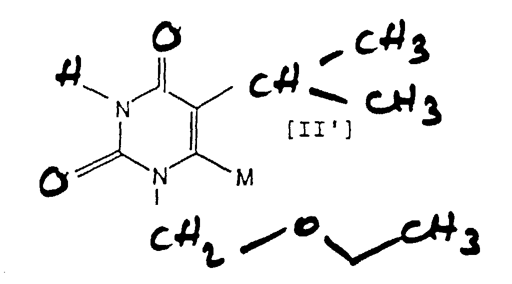 wherein
M represents an alkali metal, reacting the obtained compound of the formula II' with a compound represented by the following general formula IX; 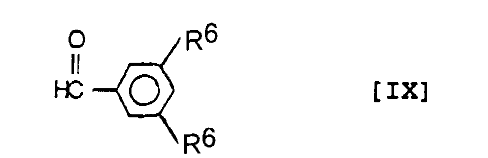 wherein R⁶ has the same meaning as defined above to obtain a compound represented by the following general formula X; 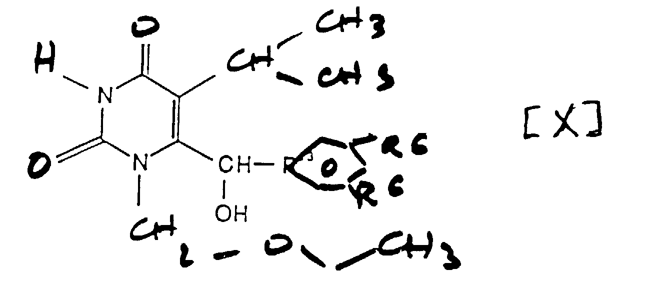 wherein R⁶ has the same meaning as defined above, and then reducing the obtained compound of the formula X using a reducing agent to obtain the compound of the formula I above.

## Patentansprüche

1. 6-substituiertes Acyclopyrimidin-Nukleosid-Derivat, das durch die folgende allgemeine Formel dargestellt ist: 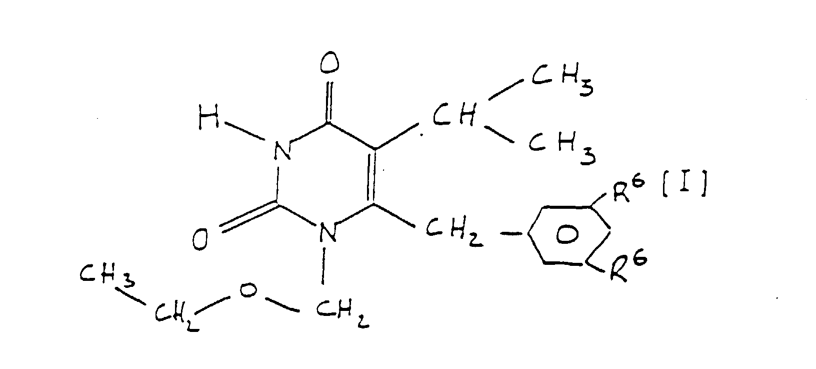 worin:
R⁶ beide entweder H oder CH₃ sind,
oder ein pharmazeutisch annehmbares Salz davon.

2. Antiretrovirales Mittel, das als Wirkstoff ein 6-substituiertes Acyclopyrimidin-Nukleosid-Derivat oder ein pharmazeutisch annehmbares Salz davon nach Anspruch 1 enthält.

3. Pharmazeutische Zusammensetzung, die ein Derivat nach Anspruch 1 oder ein pharmazeutisch annehmbares Salz davon in Verbindung mit einem pharmazeutischen Träger enthält.

4. Pharmazeutische Zusammensetzung nach Anspruch 3, welche eine effektive anti-retrovirale Wirkung aufweist.

5. Verwendung eines Derivats nach Anspruch 1 oder eines pharmazeutisch annehmbaren Salzes davon in einem Verfahren zur Herstellung einer pharmazeutischen Zusammensetzung zur Behandlung oder Verhütung von Erkrankungen, die durch eine retrovirale Infektion verursacht werden, insbesondere bei Menschen.

6. Verfahren zur Herstellung einer Verbindung, die durch die folgende allgemeine Formel dargestellt ist: 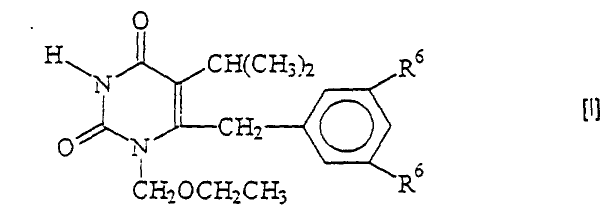 worin:
R⁶ beide entweder ein Wasserstoffatom oder CH₃ darstellen,
welches das Umsetzen einer Verbindung, die durch die folgende allgemeine Formel II dargestellt ist: 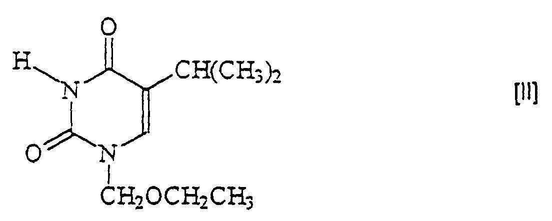 mit einer organischen Alkalimetallverbindung zum Erhalten einer Verbindung, die durch die folgende allgemeine Formel II' dargestellt ist: 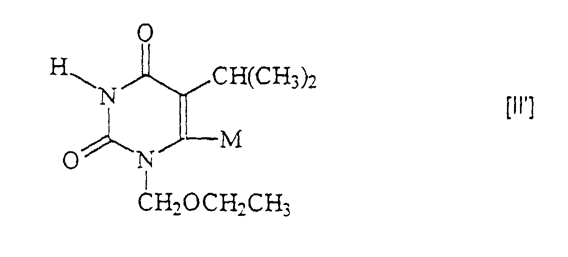 worin M ein Alkalimetall darstellt,
und anschließend das Umsetzen der erhaltenen Verbindung der Formel II' mit einer Verbindung, die durch die folgende allgemeine Formel dargestellt ist: 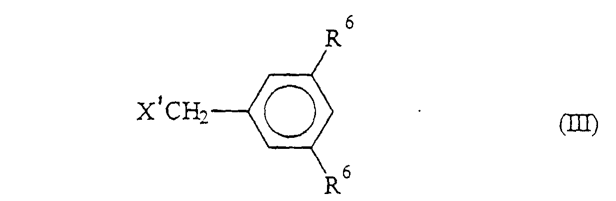 worin R⁶ die gleiche Bedeutung wie vorstehend definiert hat und X¹ ein Halogenatom, eine Arylthio- oder eine Alkoxygruppe darstellt,
zum Erhalten der Verbindung der vorstehenden Formel umfaßt.

7. Verfahren zur Herstellung einer Verbindung, die durch die folgende allgemeine Formel dargestellt ist: 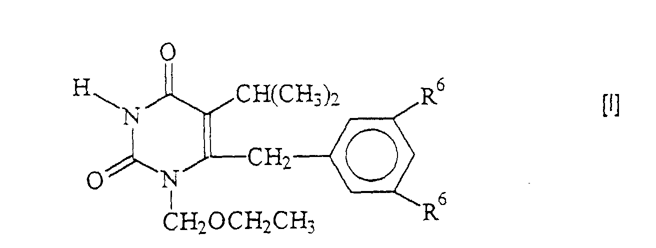 worin R⁶ die gleiche Bedeutung wie in Anspruch 1 definiert hat,
welches das Umsetzen einer Verbindung, die durch die folgende allgemeine Formel IV dargestellt ist: 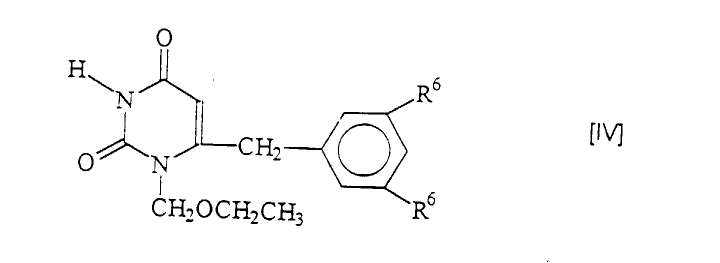 worin R⁶ die gleiche Bedeutung wie in Anspruch 1 definiert hat,
mit einer organischen Alkalimetallverbindung zum Erhalten einer Verbindung, die durch die folgende allgemeine Formel IV' dargestellt ist: 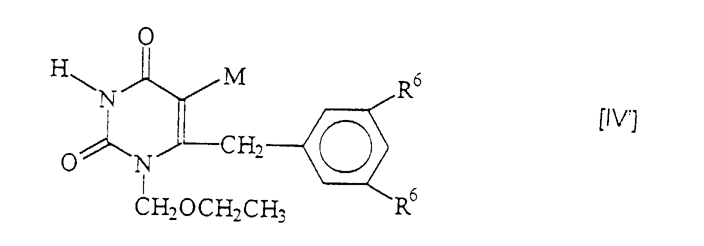 worin R⁶ die gleiche Bedeutung wie in Anspruch 1 definiert hat und M ein Alkalimetall darstellt,
und anschließend das Umsetzen der erhaltenen Verbindung der Formel IV' mit einer Verbindung, die durch die folgende allgemeine Formel V dargestellt ist:
(CH₃)₂-CH-X² [V]
worin X² ein Halogenatom darstellt,
zum Erhalten der Verbindung der vorstehenden Formel umfaßt.

8. Verfahren zur Herstellung einer Verbindung, die durch die allgemeine Formel I dargestellt ist: 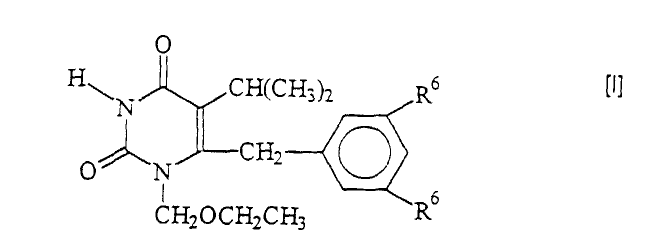 worin R⁶ die gleiche Bedeutung wie in Anspruch 1 definiert hat,
welches das Umsetzen einer Verbindung, die durch die folgende allgemeine Formel VI dargestellt ist: 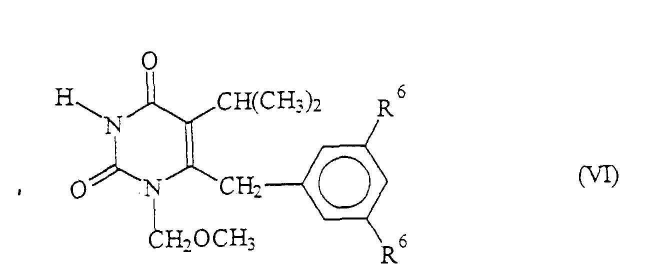 worin R⁶ die gleiche Bedeutung wie in Anspruch 1 definiert hat,
mit einer Säure zum Erhalten einer Verbindung, die durch die folgende allgemeine Formel VII dargestellt ist: 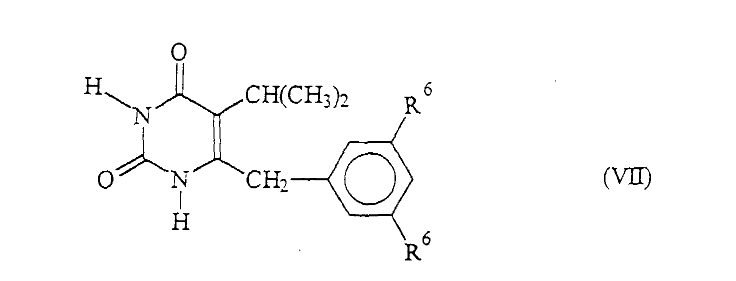 worin R⁶ die gleiche Bedeutung wie in Anspruch 1 definiert hat,
und anschließend das Umsetzen der erhaltenen Verbindung der Formel VII mit einer Verbindung, die durch die folgende allgemeine Formel VIII dargestellt ist:
CH₃-CH₂-O-CH₂-X³ [VIII]
worin X³ ein Halogenatom oder eine Sulfonyloxygruppe darstellt,
in Gegenwart einer Base zum Erhalten der Verbindung der vorstehenden Formel I umfaßt.

9. Verfahren zur Herstellung einer Verbindung, die durch die folgende allgemeine 2. Formel dargestellt ist:. 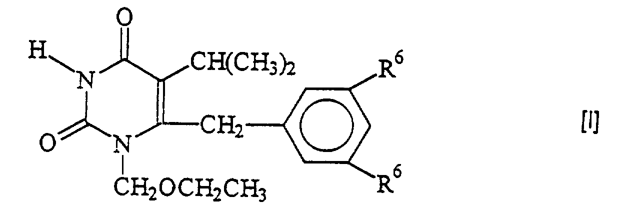 worin R⁶ die gleiche Bedeutung wie in Anspruch 1 definiert hat,
welches das Umsetzen einer Verbindung, die durch die folgende allgemeine Formel II dargestellt ist: 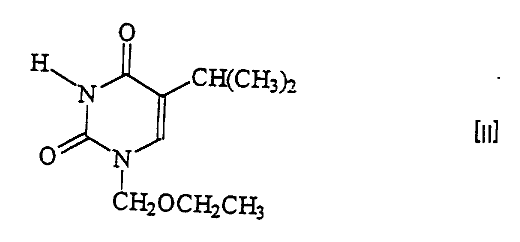 mit einer organischen Alkalimetallverbindung zum Erhalten einer Verbindung, die durch die folgende allgemeine Formel II' dargestellt ist: 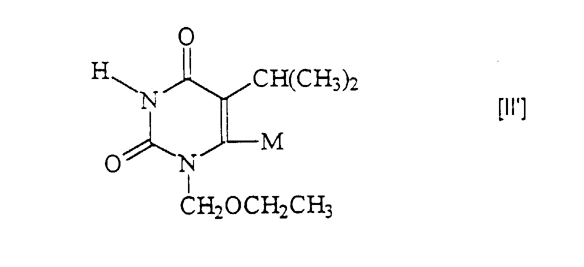 worin M ein Alkalimetall darstellt,
das Umsetzen der erhaltenen Verbindung der Formel II' mit einer Verbindung, die durch die folgende allgemeine Formel IX dargestellt ist: 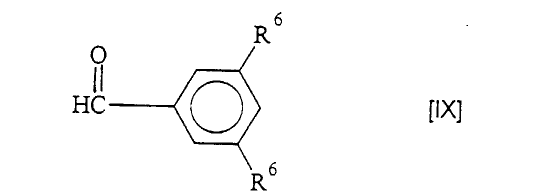 worin R⁶ die gleiche Bedeutung wie vorstehend definiert hat,
zum Erhalten einer Verbindung, die durch die folgende allgemeine Formel X dargestellt ist: 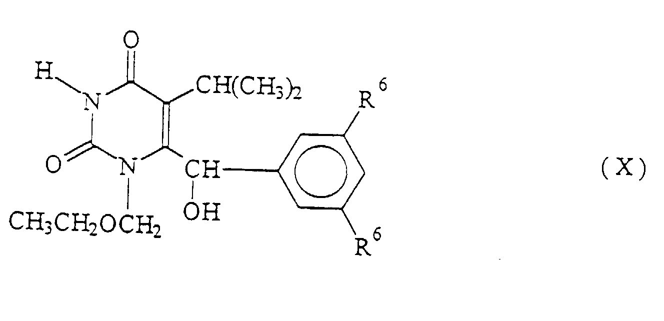 worin R⁶ die gleiche Bedeutung wie vorstehend definiert hat,
und anschließend das Reduzieren der erhaltenen Verbindung der Formel X unter Verwendung eines Reduktionsmittels zum Erhalten der Verbindung der vorstehenden Formel umfaßt.

## Revendications

1. Dérivé d'acyclopyrimidine nucléoside substitué en 6 représenté par la formule générale suivante I ; 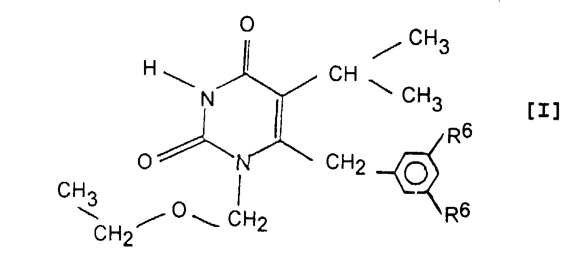 dans laquelle
R⁶ sont tous les deux H ou CH₃,
ou un sel acceptable sur le plan pharmaceutique de celui-ci.

2. Agent antirétrovirus contenant comme ingrédient actif un dérivé d'acyclopyrimidine nucléoside substitué en 6 ou un sel acceptable sur le plan pharmaceutique de celui-ci selon la revendication 1.

3. Composition pharmaceutique contenant un dérivé selon la revendication 1 ou un sel acceptable sur le plan pharmaceutique de celui-ci, associé à un véhicule pharmaceutique.

4. Composition pharmaceutique selon la revendication 3 qui possède une activité antirétrovirus efficace.

5. Utilisation d'un dérivé selon la revendication 1 ou d'un sel acceptable sur le plan pharmaceutique de celui-ci dans un procédé pour la production d'une composition pharmaceutique destinée au traitement ou à la prévention de maladies provoquées par une infection par des rétrovirus, en particulier chez les êtres humains.

6. Procédé pour la préparation d'un composé repré-senté par la formule générale suivante I ; 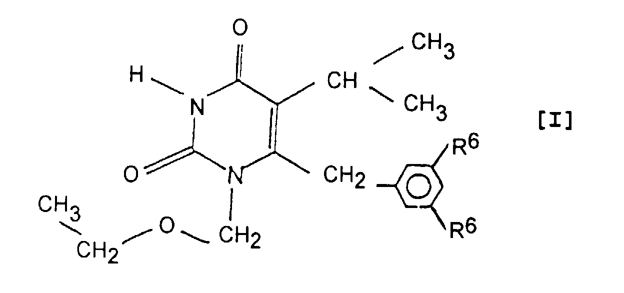 dans laquelle R⁶ représentent tous les deux soit un atome d'hydro-gène, soit CH₃,
qui consiste à faire réagir un composé représenté par la formule générale II ; 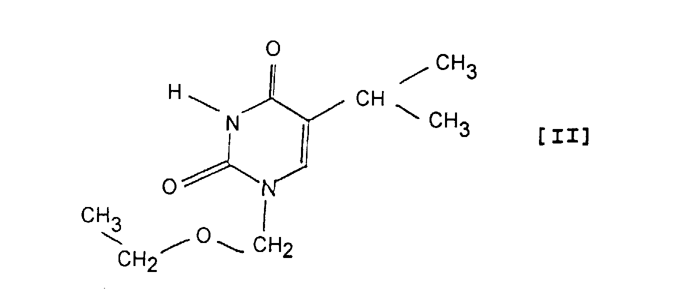 avec un composé organique d'un métal alcalin afin d'obtenir un composé représenté par la formule générale suivante II' ; 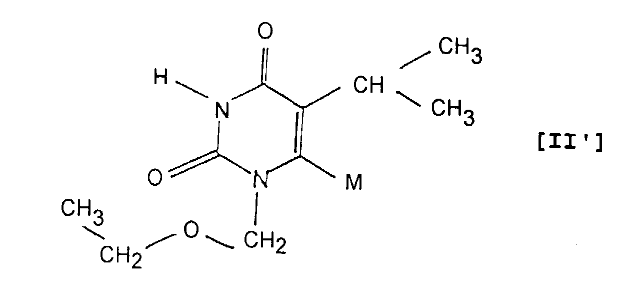 dans laquelle M représente un métal alcalin, puis à faire réagir le composé obtenu de formule II' avec un composé représenté par la formule générale suivante III ; 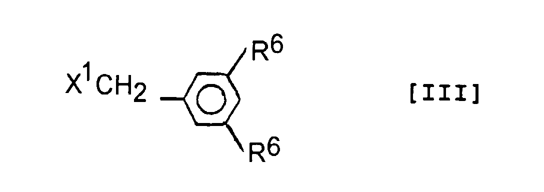 dans laquelle R⁶ possède la même signification que celle qui est définie ci-dessus et X¹ représente un atome d'halogène, un groupe arylthio ou alcoxy, afin d'obtenir le composé de formule I ci-dessus.

7. Procédé pour la préparation d'un composé repré-senté par la formule générale suivante I ; 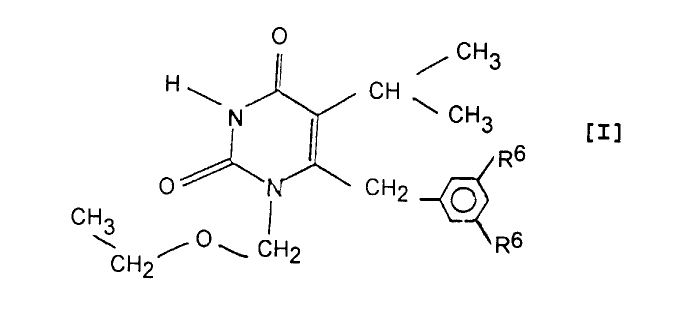 dans laquelle R⁶ possède les mêmes significations que celles qui sont définies dans la revendication 1, qui consiste à faire réagir un composé représenté par la formule générale suivante IV ; 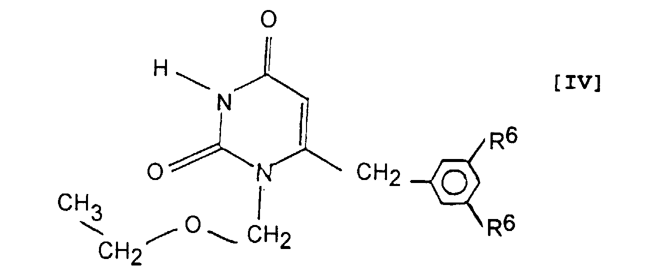 dans laquelle R⁶ possède les mêmes significations que celles qui sont définies dans la revendication 1, avec un composé organique de métal alcalin pour obtenir un composé repré-senté par la formule générale suivante IV' ; 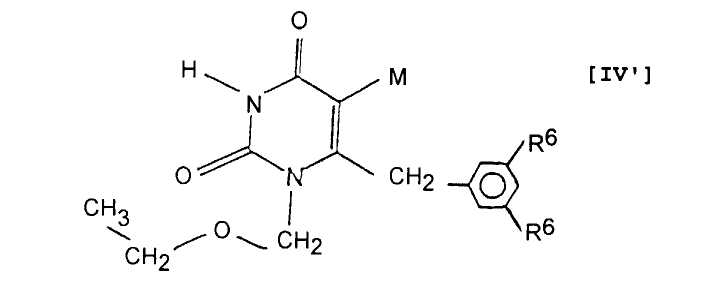 dans laquelle R⁶ possède les mêmes significations que celles qui sont définies dans la revendication 1 et M représente un métal alcalin, puis à faire réagir le composé obtenu de formule IV' avec un composé représenté par la formule générale suivante V ;
(CH₃)₂-CH-X² [V]
dans laquelle X² représente un atome d'halogène, afin d'obtenir le composé de formule I ci-dessus.

8. Procédé pour la préparation d'un composé repré-senté par la formule générale I ; 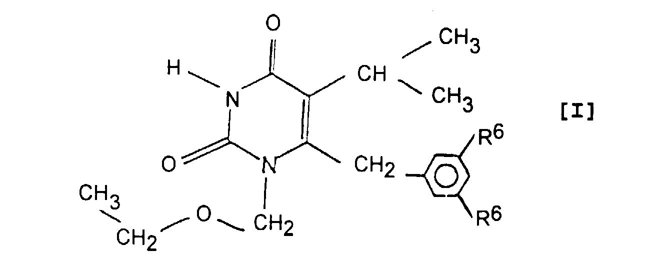 dans laquelle R⁶ possède les mêmes significations que celles qui sont définies dans la revendication 1, qui consiste à faire réagir un composé représenté par la formule générale suivante VI ; 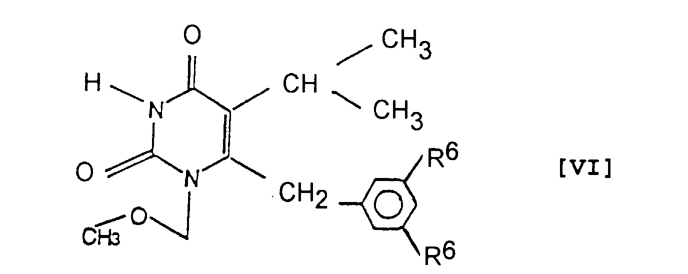 dans laquelle R⁶ possède les mêmes significations que celles qui sont définies dans la revendication 1, avec un acide pour obtenir un composé représenté par la formule générale suivante VII ; 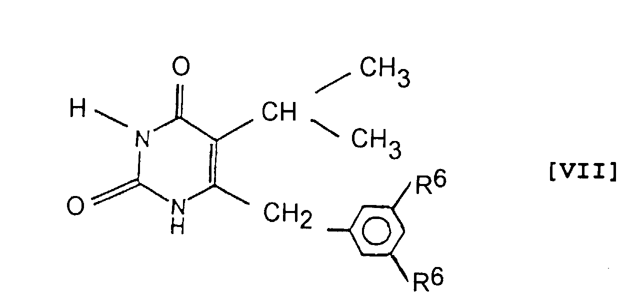 dans laquelle R⁶ possède la même signification que celle qui est définie dans la revendication 1, puis à faire réagir le composé obtenu de formule VII avec un composé représenté par la formule générale suivante VIII ;
CH₃-CH₂-O-CH₂-X³ [VIII]
dans laquelle X³ représente un atome d'halogène ou un groupe sulfonyloxy, en présence d'une base afin d'obtenir le composé de formule I ci-dessus.

9. Procédé pour la préparation d'un composé repré-senté par la formule générale suivante I ; 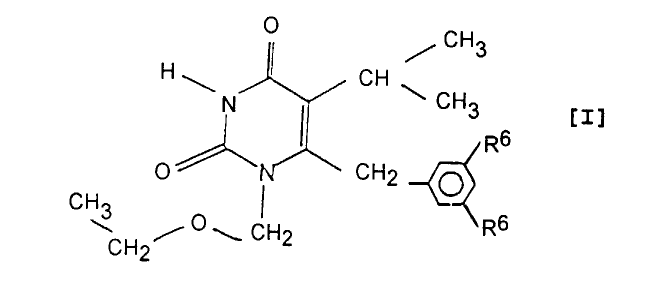 dans laquelle R⁶ possède les mêmes significations que celles qui sont définies dans la revendication 1, qui consiste à faire réagir un composé représenté par la formule générale suivante II ; 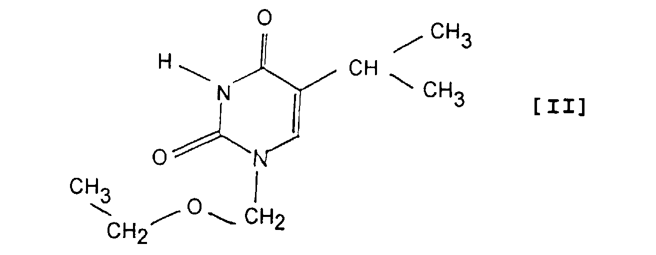 avec un composé organique de métal alcalin pour obtenir un composé représenté par la formule générale suivante II' ; 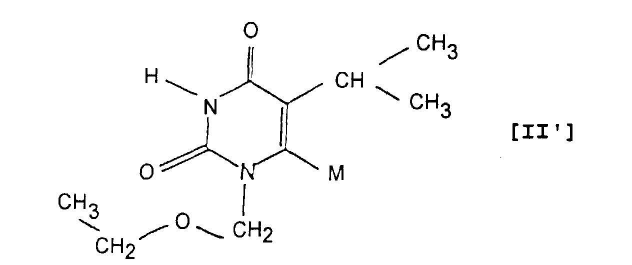 dans laquelle M représente un métal alcalin, à faire réagir le composé obtenu de formule II' avec un composé représenté par la formule générale suivante IX ; 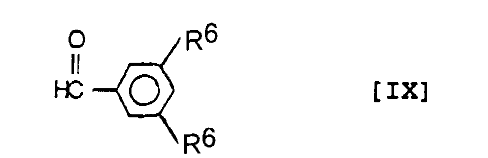 dans laquelle R⁶ possède la même signification que celle qui est définie ci-dessus afin d'obtenir le composé représenté par la formule générale suivante X ; 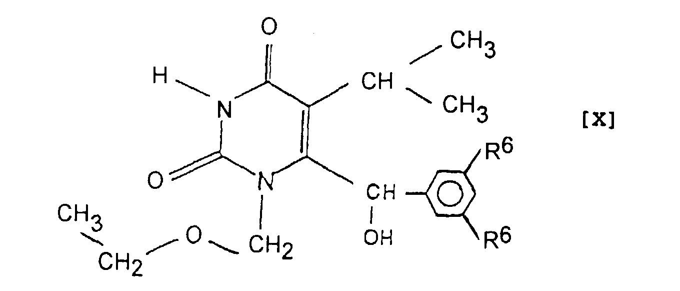 dans laquelle R⁶ possède la même signification que celle qui est définie ci-dessus, puis à réduire le composé obtenu de formule X en utilisant un agent réducteur afin d'obtenir le composé de formule I ci-dessus.
